# FASCICULE DE BREVET EUROPEEN

(11) **EP 0 553 580 B1**
(45) Date de publication et mention de la délivrance du brevet: **15.01.1997**
(21) Numéro de dépôt: 92403388.9
(22) Date de dépôt: 14.12.1992
(51) Int. Cl.: A61N 1/05

(54) **Sonde endocardiaque à barbules rabattables**
Endokardiumsonden mit klappbaren Widerhäckchen
Endocardial lead with foldable tines

(30) Priorité: 31.12.1991 FR 9116367
(43) Date de publication de la demande: 04.08.1993
(73) Titulaire: ELA MEDICAL (Société anonyme), F-92541 Montrouge Cédex (FR)
(72) Inventeur: Benel, Michel, F-92220 Bagneux (FR)
(74) Mandataire: Laget, Jean-Loup

(56) Documents cités:
- EP-A- 0 126 981
- EP-A- 0 129 875
- EP-A- 0 296 001
- EP-A- 0 329 112
- EP-A- 0 387 551
- GB-A- 1 528 073

## Description

L'invention concerne une sonde endocardiaque à barbules, notamment pour stimulateur cardiaque.

Les sondes de ce type, aussi appelées cathéters, sont utilisées pour la stimulation cardiaque en reliant un générateur de signaux, qui en général est implanté sous la peau du thorax, à la paroi interne du coeur en passant par une veine. Elles peuvent également être utilisées pour recueillir des signaux électriques en provenance du coeur. Cette sonde comporte deux extrémités: l'une appelée proximale, qui est, dans le cadre d'un stimulateur cardiaque, destinée à être connectée au générateur de signaux, l'autre appelée distale et qui est destinée à être mise en contact avec la paroi interne du muscle cardiaque.

La sonde comporte un conducteur électrique constitué par un fil métallique enroule en spirale et dispose à l'intérieur d'une gaine souple biocompatible, par exemple en caoutchouc au silicone. Le conducteur est relie à une électrode de forme annulaire ou cylindrique disposée à l'extrémité distale de la sonde.

Des barbules sont disposées au voisinage de l'extrémité distale de la sonde et elles sont destinées à venir se loger dans les trabécules s'étendant à partir de la paroi interne du coeur. L'électrode est ainsi maintenue en place contre cette paroi à l'aide des barbules prises dans les trabécules.

Il est souhaitable de réaliser des sondes' dont le diamètre total d'introduction soit le plus faible possible, ce qui est une condition absolue pour obtenir une sonde très fine. Avec des sondes fines, il est même possible de passer plusieurs sondes dans le même vaisseau sanguin.

Afin de réduire davantage l'épaisseur de la sonde à l'endroit où se trouvent les barbules, celles-ci sont généralement flexibles de manière à se rabattre contre la surface de la gaine lors de l'introduction de la sonde dans un introducteur veineux et ultérieurement lors du passage de la sonde dans une veine.

On connaît du brevet FR 2 506 596 une sonde de ce type comportant une électrode cylindrique dont l'extrémité distale est fermée par une partie bombée pleine qui fait saillie hors de la gaine et qui constitue la surface active de l'électrode. Les barbules sont venues de matière avec la gaine et elles sont disposées dans une zone de transition entre un tronçon de la gaine qui entoure l'électrode et un tronçon à diamètre réduit s'étendant vers l'arrière à partir des barbules. Les barbules sont rabattables contre la surface du tronçon de gaine à diamètre réduit, ce qui fait que l'épaisseur totale à cet endroit n'est pas supérieure au diamètre du tronçon de gaine entourant l'electrode.

L'inconvénient de cette sonde est que les barbules se trouvent par cette construction même relat.ivement eloignées de l'extrémité distale de l'électrode qui pour cette raison est moins bien retenue en place.

Le but de l'invention est de proposer une sonde à barbules, permettant l'emplacement des barbules à proximité de la surface extrême active de l'électrode, tout en réduisant au maximum le diamètre d'introduction de la sonde. Grâce à cette disposition des barbules, la retenue en place de l'électrode contre la paroi interne du coeur se trouve améliorée.

L'invention a pour objet une sonde endocardiaque, notamment pour stimulateur cardiaque, du type constituée par un conducteur électrique enroulé en spirale et disposé à l'intérieur d'une gaine souple, ce conducteur étant relié à une electrode disposée à l'extremité distale de la sonde et destinée à être placée contre une paroi interne du coeur et retenue en place par des barbules disposées sur la périphérie de la sonde, au voisinage de l'extrémité distale de celle-ci, et s'étendant vers l'arrière de la sonde, ces barbules étant flexibles afin d'être rabattues contre la surface de la sonde lors de l'introduction de celle-ci dans un introducteur veineux tubulaire et ultérieurement dans une veine, caracterisée en ce que la sonde comporte au voisinage de son extrémité distale, des évidements allongés disposés de façon à former des logements dans lesquels les barbules sont sur toute leur longeur au moins partiellement escamotables.

Selon d'autres caractéristiques de l'invention :
- les évidements sont des fentes ménagées dans un manchon dispose autour de l'extrémité distale de la sonde;
- le manchon est en une seule pièce avec l'electrode, et le manchon proprement dit est recouvert d'un revêtement électriquement isolant sur la partie non active de l'electrode;
- le manchon est une pièce rapportée;
- plusieurs fentes sont longitudinalement alignées sur la surface du manchon;
- plusieurs fentes sont décalées les unes par rapport aux autres sur la périphérie du manchon;
- les barbules sont venues de matière avec un fourreau surmoulé ou collé sur l'extrémité distale de la gaine, et le fourreau s'étend vers l'électrode à partir d'un interstice entre la gaine et le manchon;
- le fourreau est en caoutchouc au silicone ou autre matériau biocompatible;
- la pièce constituée par l'électrode et le manchon est en titane ou en carbone, ou autre matériau électriquement conducteur;
- le manchon est en platine-iridium ou autre matériau radio-opaque.

L'invention sera maintenant décrite plus en détail en référence au dessin annexé sur lequel :
- la Figure 1 est une vue en élévation, partiellement en coupe axiale, de l'extrémité distale d'une sonde à barbules suivant l'invention après l'introduction de la sonde dans un introducteur veineux;
- La Figure 2 est une vue analogue à celle de la Figure 1, montrant la sonde et la position de ses barbules, avant l'introduction de la sonde dans l'introducteur veineux et après la mise en place de la sonde dans le coeur;
- La Figure 3 est une vue en élévation, partiellement en coupe axiale, de l'extrémité distale d'une sonde selon un autre mode de réalisation de l'invention.

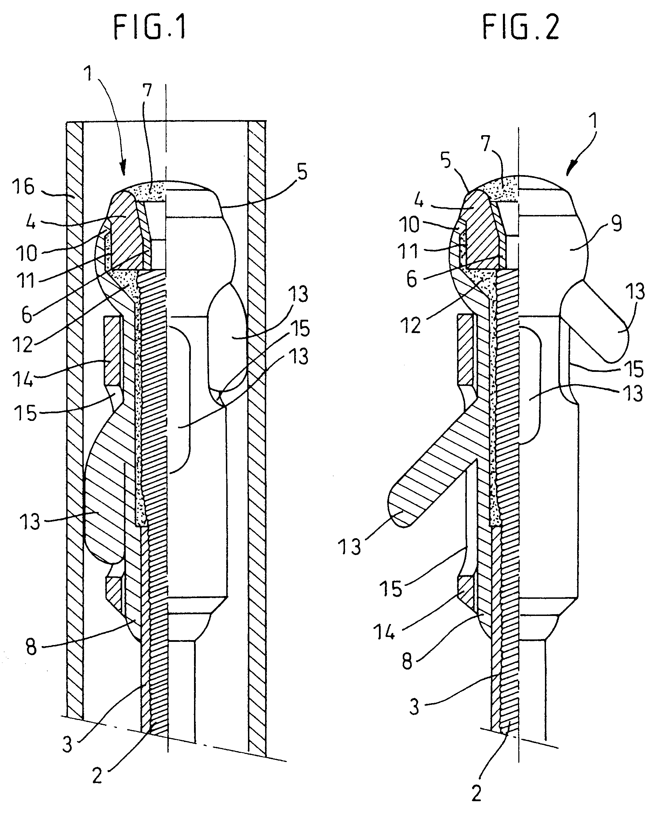

Les Figures 1 et 2 montre une sonde 1 à barbules selon un premier mode de réalisation de l'invention.

La sonde 1 comporte un conducteur électrique 2 enroulé en spirale et disposé à l'intérieur d'une gaine 3.

Le conducteur 2 peut être fabriqué par exemple en un alliage comprenant du nickel, du cobalt, du chrome et du molybdène, la gaine étant en un matériau biocompatible tel que le caoutchouc au silicone ou le polyuréthane.

Le conducteur 2 est relié à une électrode annulaire 4 disposé à l'extrémité distale de la sonde et qui présente une surface 5 active à angles arrondis destinée à être disposée en contact avec la paroi interne du coeur. La partie distale de la spirale du conducteur 2 est dans l'exemple illustre maintenue serrée contre la face interne cylindrique de l'électrode à l'aide d'un axe creux 6. L'axe creux 6 assure un bon contact entre les ultimes spires du conducteur et l'électrode afin d'éviter des micro-coupures entre ces deux éléments.

L'ouverture définie par la face interne de l'electrode annulaire est bouchée par une colle 7, comportant par exemple du silicone, et qui forme ainsi une étanchéité empêchant les fluides de pénétrer par cet.te ouverture dans l'intérieur de la sonde.

L'extrémité distale de la sonde est en outre pourvue d'un fourreau 8 reliant la gaine 3 à l'electrode 4. Le fourreau 8 est fixé autour de l'extrémité de la gaine. L'extrémité distale 9 du fourreau en contact avec l'électrode 4 a une forme générale sphérique et la périphérie de l'électrode est tenue par le rebord du fourreau.

Le fourreau 8 est dans l'exemple illustré sur les figures une pièce collée ou surmoulée sur la gaine 3.

La partie avant active 5 de l'électrode 4 est par ailleurs délimité vers l'arrière par un épaulement 10 s'étendant vers le centre et la partie arrière ou proximale 11 de l'électrode a une forme cylindrique.

La partie cylindrique 11 de l'électrode 4 est fixée dans le fourreau 8 à l'aide d'une colle électriquement isolante 12 injectée dans l'interstice forme entre d'une part la paroi interne de la partie cylindrique 11 du fourreau et le conducteur hélicoïdal 2, et d'autre part entre la paroi interne de la partie sphérique 9 du fourreau et la face externe de la partie cylindrique 11 de l'électrode 4.

Le fourreau 8 porte sur sa surface externe des barbules 13. Ces barbules peuvent être surmoulées sur le fourreau, mais elles sont avantageusement venues de matière avec celui-ci, comme montre sur les figures 1 et 2. Le fourreau 8 est en un matériau biocompatible, de préférence du même matériau que la gaine 3.

Les barbules 13 sont régulièrement disposées sur la surface du fourreau 8 et s'étendent vers l'arrière de la sonde.

Le fourreau 8 est disposé à l'intérieur d'un manchon radio-opaque 14 pourvu d'évidements sous forme de fentes 15 à travers lesquelles les barbules 13 font saillie vers l'extérieur. Les fentes 15 ont un tel contour qu'elles forment des logements dans lesquels des barbules 13 peuvent au moins partiellement s'escamoter lors de l'introduction de la sonde 1 dans un introducteur veineux 14 schématiquement illustré à la figure 1.

Plusieurs barbules 13 et fentes associées 15 peuvent être longitudinalement alignées sur la surface du manchon 14 ou décalées les unes par rapport aux autres sur la surface du manchon, ou bien présenter une combinaison de ces deux dispositions.

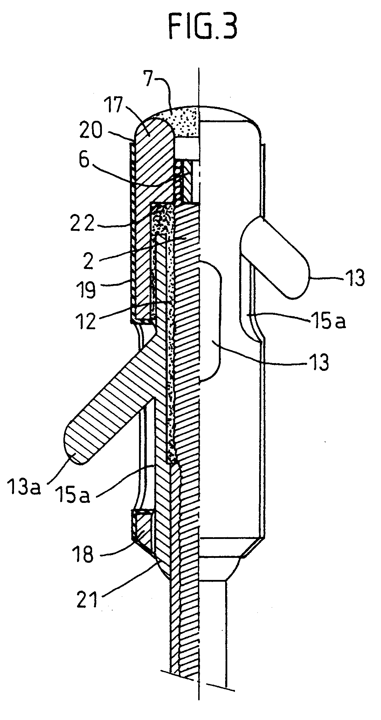

La figure 3 montre un autre mode de réalisation de l'invention. Des éléments identiques portent les mêmes numéros de référence et ils ne seront pas décrits davantage.

La différence essentielle par rapport au premier mode de réalisation est que l'électrode 17 et le manchon 18 sont selon le deuxième mode de réalisation en une seule pièce. Le manchon proprement dit est recouvert par un revêtement électriquement isolant 19 alors que la surface active 20 de l'electrode 17 est dépourvue de ce revêtement.

Le diamètre du fourreau 21 est constant sur toute la longueur de celui-ci. Le manchon 18 est pourvu d'un épaulement interne 22 s'étendant vers le centre de manière à former une surface de portée circulaire disposée contre la surface d'extrémité du fourreau 21.

Comme dans le premier mode de réalisation, le manchon 18 comporte des fentes 15a à travers lesquelles s'etendent les barbules flexibles 13a reliées au fourreau 21.

Par ailleurs, l'électrode 17 de cette sonde est bouchée par de la colle 7, et une colle isolante est injectée dans l'interstice entre le conducteur 2 et la paroi interne du fourreau 21.

La sonde obtenue selon les deux exemples non limitatifs de l'invention présente ainsi un diamètre plus faible que celui des sondes connues du même type, grâce au fait que les barbules 13 peuvent être au moins partiellement escamotées dans des évidements 15 ménagés sur la périphérie de la sonde, à proximité de l'extrémité distale de celle-ci.

## Revendications

1. Sonde endocardiaque, notamment pour stimulateur cardiaque, du type constituée par un conducteur électrique (2) enroulé en spirale et disposé à l'intérieur d'une gaine souple (3), ce conducteur (2) étant relié à une électrode (4; 17) disposée à l'extrémité distale de la sonde (1) et destinée à être placée contre une paroi interne du coeur et retenue en place par des barbules (13; 13a) disposées sur la périphérie de la sonde (1), au voisinage de l'extrémité distale de celle-ci, et s'étendant vers l'arrière de la sonde, ces barbules (13; 13a) étant flexibles afin d'être rabattues contre la surface de la sonde lors de l'introduction de celle-ci dans un introducteur veineux tubulaire (16) et ultérieurement dans une veine, caractérisée en ce que la sonde (1) comporte au voisinage de son extrémité distale, des évidements allongés (15; 15a) disposés de façon à former des logements dans lesquels les barbules (13; 13a) sont sur toute leur longeur au moins partiellement escamotables.

2. Sonde suivant la revendication 1, caractérisée en ce que les évidements sont des fentes (15; 15a) ménagées dans un manchon (14; 18) disposé autour de l'extrémité distale de la sonde (1).

3. Sonde suivant la revendication 2, caractérisée en ce que le manchon (18) est en une seule pièce avec l'électrode(17), et en ce que le manchon proprement dit est recouvert d'un revêtement (19) électriquement isolant sur la partie non active de l'électrode;

4. Sonde suivant la revendication 2, caractérisée en ce que le manchon (14) est une pièce rapportée.

5. Sonde suivant l'une quelconque des revendications 2 à 4, caractérisée en ce que plusieurs fentes (15; 15a) sont longitudinalement alignées sur la surface du manchon (14; 18).

6. Sonde suivant l'une quelconque des revendications 2 à 5, caractérisée en ce que plusieurs fentes sont décalées les unes par rapport aux autres sur la périphérie du manchon (14; 18).

7. Sonde suivant l'une quelconque des revendications précédentes, caractérisée en ce que les barbules (13; 13a) sont venues de matière avec un fourreau (8; 21) surmoulé ou collé sur l'extrémité distale de la gaine (3), en ce que le fourreau (8; 21) s'étend vers l'électrode (4; 17) à partir d'un interstice entre la gaine (3) et le manchon (14; 18).

8. Sonde suivant la revendication 7, caractérisée en ce que le fourreau (8; 21) est en caoutchouc au silicone ou autre matériau biocompatible.

9. Sonde suivant l'une quelconque des revendications 2, 3, 5 à 8, caractérisée en ce que la pièce constituée par l'électrode (17) et le manchon (18) est en titane ou en carbone, ou autre matériau électriquement conducteur.

10. Sonde suivant l'une quelconque des revendications 2, 4, 5 à 8, caractérisée en ce que le manchon (14) est en platine-iridium ou autre matériau radio-opaque.

## Claims

1. An endocardiac lead, in particular for a cardiac pacemaker, of the type formed by an electrical conductor (2) wound in a coil and arranged within a flexible sheath (3), said conductor (2) being connected to an electrode (4; 17) arranged at the distal end of the lead (1) and intended to be placed against an inner wall of the heart and held in place by fins (13; 13a) arranged on the periphery of the lead (1), in the vicinity of the distal end thereof, and extending towards the rear of the lead, these fins (13; 13a) being flexible in order to be folded back against the surface of the lead when it is being introduced into a tubular venous introducer (16) and later into a vein, characterised in that the lead (1) comprises elongated recesses (15; 15a) in the vicinity of its distal end, arranged so as to form housings in which the fins (13; 13a) can be retracted at least partially over their entire length.

2. A lead according to Claim 1, characterised in that the recesses are slots (15; 15a) made in a sleeve (14; 18) arranged around the distal end of the lead (1).

3. A lead according to Claim 2, characterised in that the sleeve (18) is of one piece with the electrode (17), and in that the sleeve proper is covered with an electrically insulating covering (19) on the non-active part of the electrode.

4. A lead according to Claim 2, characterised in that the sleeve (14) is a loose piece.

5. A lead according to any one of Claims 2 to 4, characterised in that a plurality of yslots (15; 15a) are longitudinally aligned on the surface of the sleeve (14; 18).

6. A lead according to any one of Claims 2 to 5, characterised in that a plurality of slots are offset relative to each other on the periphery of the sleeve (14; 18).

7. A lead according to any one of the preceding claims, characterised in that the fins (13; 13a) are integral with a cover (8; 21) moulded on or glued to the distal end of the sheath (3), and in that the cover (8; 21) extends towards the electrode (4; 17) from a gap between the sheath (3) and the sleeve (14; 18).

8. A lead according to Claim 7, characterised in that the cover (8; 21) is made of silicone rubber or other biocompatible material.

9. A lead according to any one of Claims 2, 3, 5 to 8, characterised in that the part formed by the electrode (17) and the sleeve (18) is made of titanium or carbon, or another electrically conductive material.

10. A lead according to any one of Claims 2, 4, 5 to 8, characterised in that the sleeve (14) is made of platinum-iridium or of another radio-opaque material.

## Patentansprüche

1. Endokardiumsonde, insbesondere für Herzschrittmacher, einer durch einen spiralförmig gewickelten und im Inneren einer biegsamen Ummantelung (3) angeordneten elektrischen Leiter (2) gebildeten Bauart, wobei der Leiter (2) mit einer am entfernten Ende der Sonde (1) angeordneten Elektrode (4; 17) verbunden ist, die dazu bestimmt ist, an eine innere Herzwandung angelegt zu werden und mittels am Umfang der Sonde (1) in der Nähe ihres entfernten Endes vorgesehener und sich zu einem rückwärtigen Teil der Sonde erstreckender Widerhäkchen (13; 13a) fixiert zu werden, wobei die Widerhäkchen (13; 13a) beweglich sind, um beim Einführen der Sonde in eine röhrenförmige Venen-Einführvorrichtung (16), und letzlich in eine Vene, gegen die Oberfläche der Sonde geklappt zu werden,
**dadurch gekennzeichnet, daß**
die Sonde (1) in der Nähe ihres entfernten Endes längliche Aussparungen (15; 15a) aufweist, die Aufnahmen bilden, in denen die Widerhäkchen (13; 13a) in ihrer gesamten Länge zumindest teilweise einklappbar sind.

2. Sonde nach Anspruch 1,
**dadurch gekennzeichnet, daß**
es sich bei den Aussparungen um Langlöcher (15; 15a) handelt, die in einer um das entfernte Ende der Sonde (1) angeordneten Hülse (14; 18) vorgesehen sind.

3. Sonde nach Anspruch 2,
**dadurch gekennzeichnet, daß**
die Hülse (18) einstückig mit der Elektrode (17) ausgebildet ist und daß die Hülse im elektrisch inaktiven Abschnitt der Elektrode von einer elektrisch isolierenden Umhüllung (19) umgeben ist.

4. Sonde nach Anspruch 2,
**dadurch gekennzeichnet, daß**
die Hülse (14) ein Aufsatzstück ist.

5. Sonde nach einem der Ansprüche 2 bis 4,
**dadurch gekennzeichnet, daß**
mehrere Langlöcher (15; 15a) in Längsrichtung an der Oberfläche der Hülse (14; 18) ausgerichtet sind.

6. Sonde nach einem der Ansprüche 2 bis 5,
**dadurch gekennzeichnet, daß**
mehrere Langlöcher am Umfang der Hülse (14; 18) zueinander versetzt angeordnet sind.

7. Sonde nach einem der vorhergehenden Ansprüche,
**dadurch gekennzeichnet, daß**
die Widerhäkchen (13; 13a) einstückig mit einer Manschette (8, 21) ausgebildet sind, die dem entfernten Ende der Ummantelung (3) aufgeformt oder angeklebt ist, und daß sich die Manschette (8; 21) ausgehend von einem Spalt zwischen der Ummantelung (3) und der Hülse (14; 18) in Richtung auf die Elektrode (4, 17) erstreckt.

8. Sonde nach Anspruch 7,
**dadurch gekennzeichnet, daß**
die Manschette (8; 21) aus Kautschuk oder Silikon oder einem anderen biologisch verträglichen Material besteht.

9. Sonde nach einem der Ansprüche 2, 3, 5 bis 8,
**dadurch gekennzeichnet, daß**
das von der Elektrode (17) und der Hülse (18) gebildete Bauteil aus Titan oder Kohlenstoff oder einem anderen elektrisch leitfähigen Material besteht.

10. Sonde nach einem der Ansprüche 2, 4, 5 bis 8,
**dadurch gekennzeichnet, daß**
die Hülse (14) aus Platin-Iridium oder einem anderen strahlungsundurchlässigen Material besteht.
